# EUROPEAN PATENT SPECIFICATION

(11) **EP 1 283 672 B1**
(45) Date of publication and mention of the grant of the patent: **17.11.2004**
(21) Application number: 01935147.7
(22) Date of filing: 08.05.2001
(51) Int. Cl.: A01N 33/02, C07C 215/00, C07C 255/00, C07D 317/22

(54) **ANTITUBULIN ASSEMBLY AND CELL GROWTH INHIBITOR DENOMINATED "DIOXOSTATIN"**
ANTITUBULINANORDNUNG UND ZELLENWACHSTUMSHEMMSTOFF "DIOXOSTATIN"
ENSEMBLE ANTITUBULINE ET INHIBITEUR DE CROISSANCE CELLULAIRE APPELE "DIOXOSTATINE"

(30) Priority: 09.05.2000 US 202770 P
(43) Date of publication of application: 19.02.2003
(73) Proprietor: Arizona Board of Regents, acting for and on behalf of Arizona State University, Tempe, AZ 85287 (US)
(72) Inventor: PETTIT, George, R., Paradise Valley, AZ 85253 (US); LIPPERT, John, W., III, Schenectady, NY 12303 (US)
(74) Representative: Thomas, Philip John Duval
(86) International application number: PCT/US2001/014790
(87) International publication number: WO 2001/084929

(56) References cited:
- WO-A1-01/19794
- US-A- 5 525 632
- SHIRAI, RYUICHI ET AL: "Asymmetric synthesis of antimitotic combretadioxolane with potent antitumor activity against multi-drug resistant cells" BIOORGANIC & MEDICINAL CHEMISTRY LETTERS (1998), 8(15), 1997-2000 , XP004137173
- SHIRAI, RYUICHI ET AL: "Synthesis of conformationary restricted combretastatins" HETEROCYCLES (1997), 46, 145-148 , XP002237062
- DATABASE MEDLINE [Online] PETTIT G.R. ET AL.: 'Antineoplastic agents 442. Synthesis and biological activities of dioxostatin', XP002940833 Retrieved from STN Database accession no. 2001256019 & ANTI-CANCER DRUG DESIGN vol. 15, no. 5, October 2000, pages 361 - 371, XP002940833
- DATABASE EMBASE [Online] PETTIT G.R. ET AL.: 'Antineoplastic agents 379. Synthesis of phenstatin phosphate', XP002940809 Retrieved from STN Database accession no. 1998162066 & JOURNAL OF MEDICINAL CHEMISTRY vol. 41, no. 10, 07 May 1998, pages 1688 - 1695
- DATABASE BIOSIS [Online] TAHIR S.K. ET AL.: 'Rapid colchicine competition-binding scintillation proximity assay using biotin-labeled tubulin', XP002941888 Retrieved from STN Database accession no. 2000:393158 & BIOTECHNIQUES vol. 29, no. 1, July 2000, pages 156 - 160

## Description

### Introduction

This invention relates generally to the field of chemotherapy and more particularly to the recovery and structural elucidation of a new inhibitor of microtuble assembly (IC₅₀ 0.59µM) and cell growth denominated "dioxostatin".

This research was funded in part by the Outstanding Investigation Grant CA 44344-01-11 awarded by the Division of Cancer Research Treatment, National Cancer Institute, DHHS. Accordingly, the United States government may have certain rights to this invention.

### Background Of The Invention

The elucidation and isolation of agents from the African bush willow *Combretum caffrum* first identified combretastatin A-4 as described in U.S. Patent No. 4,996,237 which issued to G.R. Pettit et al., on February 26, 1991. Other early efforts to develop a combretastatin A-4 prodrug are described in U.S. Patent No. 5,561,122, which issued to G.R. Pettit on October 1, 1996. The general background information from each of these patents is incorporated herein by this reference thereto.

The potent cancer cell growth and tubulin assembly inhibitor combretastatin A-4 was originally isolated from the African tree *Combretum caffrum* (Combretaceae) circa 1985 and has been undergoing preclinical development since that time. However, because of the very limited aqueous solubility of the phenol and its alkali metal salts, drug formulation attempts gave unsatisfactory results.

The African willow tree *Combretum caffrum* Kuntze (*Combretaceae*) has proven to be a very productive source of cancer cell growth (murine P388 lymphocytic leukemia) inhibitory stilbenes, bibenzyls and phenanthrenes. Since 1979 promising leads have been pursued which were focused on the two most active (inhibition of cancer cell growth and polymerization of tubulin (*Id*.)) constituents, namely combretastatin A-1 **(1a)** and A-4 **(1b)** (*Id*.) (*See*, Figure 1). Of these, combretastatin A-4 **(1b)** has reached the most advanced stage of predinical development as the very soluble prodrug 1d. Meanwhile other research groups have also been further extending structure/activity relationships (hereinafter referred to as "SAR") among the combretastatins and related stilbenes.

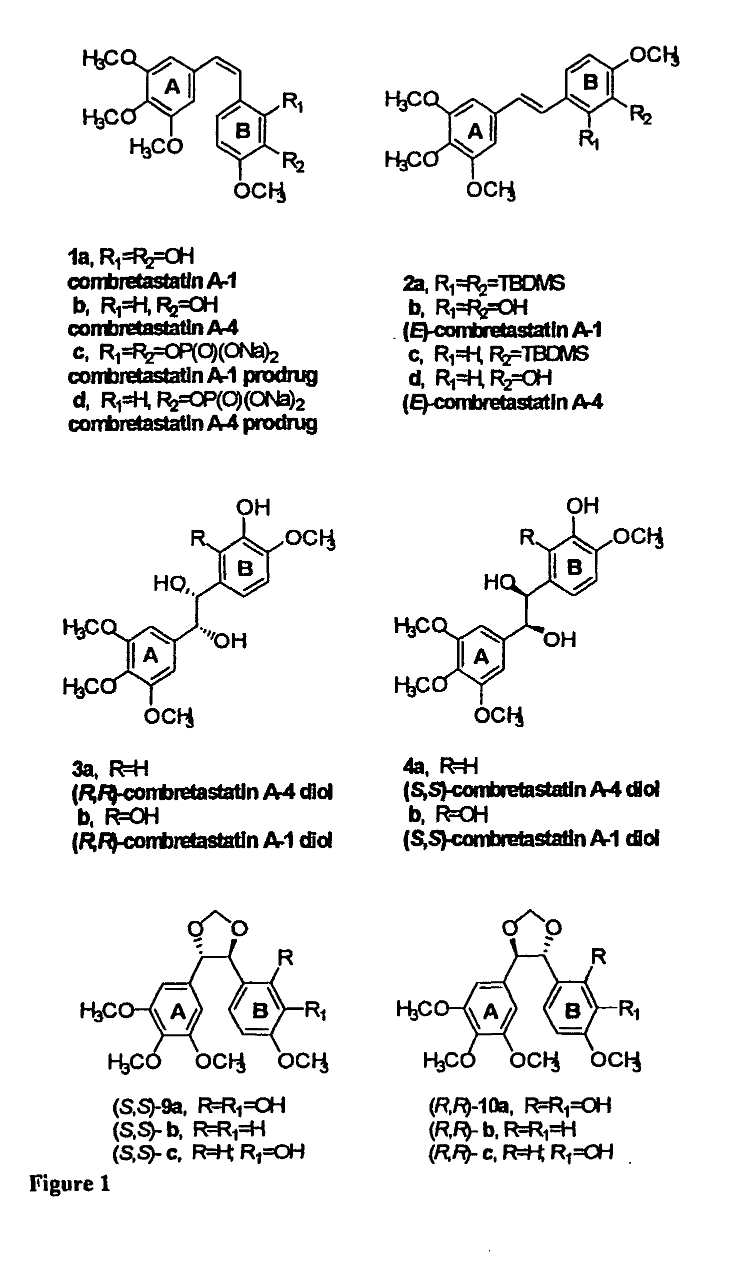

In 1987 and 1989 respectively, combretastatin A-1 **(1a)** and A-4 **(1b)** were reported which structurally resembled the antimitotic and cell growth inhibitors colchicine and podophyllotoxin, and which represent two of the most promising antineoplastic constituents of *C. caffrum*. The monophenol **1b** proved to be a potent inhibitor of microtubule assembly (IC₅₀ 2-3 µM) and exhibited an ED₅₀ of 0.0003 µg/mL against the murine P388 leukemia cell line, while the diphenol **1a,** while equally effective as an inhibitor of microtubule assembly (IC₅₀ 2-3 µM), was much less potent as an inhibitor of P388 cell growth (ED₅₀ 0.2 µg/mL).

Presumably the reduced cytotoxicity to cancer cells shown by the diphenol **1a** is due to the presence of its vicinal phenolic groups, which can be easily oxidized to the 1,2-quinone. Such an interpretation was supported by the fact that acetylation of the hydroxyl groups enhanced cytotoxicity 10-fold, while significantly reducing the in vitro inhibition of tubulin polymerization and colchicine binding. In spite of these superficial negative aspects, some biological properties exhibited by diphenol **1a** make it attractive. For example diphenol **1a** may be the most potent antagonist of colchicine binding known, with nearly a 99% inhibition at equal concentrations, In addition, diphenol **1a** was found to be more potent than monophenol **1b** in its ability to increase intracellular daunorubicin concentrations in MDR (multidrug resistant) cancer cell lines. Most importantly, tubulin-binding stilbenes **1a** and **1b** selectively elicit irreversible vascular shutdown within solid tumors. The degree of reduction ranged from 50% with **1a** to 70% with **1b,** while the combretastatin A-4 prodrug **(1d),** a sodium phosphate derivative of monophenol **1b,** induced a complete vascular shutdown within metastatic tumors at doses one-tenth of the maximum tolerated dose. Those very encouraging results indicated that the A-series of combretastatin stilbenes should be further investigated in respect to their antiangiogenic and other anticancer properties. Hence a structure- activity relationship (SAR) study of diphenol **1a** was initiated to parallel a long-term investigation of the combretastatin A-4 prodrug **(1d).**

The formation of new blood vessels, known as "angiogenesis" (neovascularisation), is controlled by a very complex series of biochemical interactions involving a large number of angiogenic factors ranging from various cytokines (e.g. IL-1) and growth factors (e.g., GM-CSF) to serine proteases (e.g. urokinase). In general, normal angiogenesis involves the activation and transport of endothelial cells from already formed blood vessels to new locations. Normally that transition takes from three months to a year except in wound healing and in certain stages of female reproductive biology. When the angiogenic control mechanisms fail, the results lead to a wide range of human disease categories, such as cancer psoriasis, hemangioma, atherosclerotic plaque, diabetic and macular retinopathy, neovascular glaucoma, and vascular adhesions following surgery.

Because the African bush willow (*Combretum caffrum*) constituents combretastatins A-1 **(1a)** and A-4 **(1b)** were isolated and designated as well as their phosphate prodrug derivatives **(1c,d)** which displayed very promising antineoplastic, cancer antiangiogenesis, and other important biological activities. Recently extended SAR investigations of these *cis*-stilbenes have been conducted. Indeed, combretastatin A-4 prodrug **(1d)** has been undergoing a series of phase I human cancer clinical trials since November 1998.

Previous SAR analyses of the combretastatin A-4 series have indicated that the *cis* configuration of the stilbene unit is the most important factor for inhibition of cancer cell growth and inhibiting tubulin assembly. With the corresponding (*E*)-stilbenes, the cancer cell growth inhibitory and antitubulin activity is greatly reduced from that exhibited by the corresponding (*Z*)-isomers. Initially, both the *trans*-isomers **2b** and **2d** were found to be moderately active as cancer cell growth inhibitors. Later studies using *trans*-stilbene **2d** revealed that freshly prepared solutions in dimethyl sulfoxide were inactive and only gained activity with the passage of time suggesting that the *trans*-isomers were slowly converted to the *cis* active isomers.

### Brief Summary of the Invention

A high yield regioselective synthesis of *E*-combretastatin A-1 **(2b)** was completed using MOM protection and a Wadsworth-Emmons reaction as key steps. In turn, (*E*)-stilbene **15** was converted by convenient syntheses to both (*S*,*S*)-and (*R*,*R*)-1,3-dioxolanes **9a** and **10a**. A Sharpless asymmetric dihydroxylation reaction was employed for preparation of intermediates (*S*,*S*)-**16** and (*R*,*R*)-**17**. The (4*S*,5*S*)-4-(2',3'-dihydroxy-4'-methoxyphenyl)-5-(3",4",5"-trimethoxyphenyl)-1,3-dioxolane (**9a**) was found to be a highly potent inhibitor of microtubule assembly (IC₅₀ 0.59 µM) and was designated "dioxostatin". Conversion to sodium phosphate **21** (P388 lymphocytic leukemia cell line ED₅₀ 0.2 µg/mL) provided a very useful water-soluble prodrug. Accordingly, a principal object of the present invention is to provide a new and unexpectedly active inhibitor of microtuble assembly and cell growth by the synthesis and structure determination of a derivative of combretastatin herein denominated "dioxostatin".

Another object of the present invention is the formulation of a water soluble chemotherapeutic agent which is effective in the treatment of leukemia and other cancerous disorders associateable positive results against the NCI P388 cell line.

These and still further objects as shall hereinafter appear are readily fulfilled by the present invention in a remarkably unexpected manner as will be readily discerned from the following detailed description of an exemplary embodiment thereof especially when read in conjunction with the accompanying schemes.

### Statement of the Invention

The present invention is defined in the accompanying claims.

### Description of the Preferred Embodiment

Recently the (*E*)-isomer of combretastatin A-4(2d) was converted to chiral diols (R,R)-3a and (S,S)-4a, where the (*E*)-olefin unit was replaced by a freely rotating *sp*^{*3*}-hybridized chiral ethanediol. These experiments have now been extended to include the (*E*)-isomer of combretastatin A-1 generating chiral diols (R,R)-3b and (S,S)-4b. Asymmetric dihydroxylation (See: Kolb, H. C.; VanNieuwenhze, M.S.; Sharpless, K. B. Chem. Rev. 1994, *94*, 2483-2547); with AD-mix-α afforded (*S*_{*1*}*S*)-diol 5. Photoisomerization of the (*E*)-stilbene was prevented using an alumiunium foil wrapped reaction vessel.

As a check that the correct chirality was being induced in the preparation of diols (R,R)-3b and (S,S)-4b, a single crystal X-ray structure determination was conducted on one of the intermediate silyl protected products formed in the AD-mix reactions. Thus, treatment of the *trans*-stilbene 2a with AD-mix-α yielded diol 5, which was expected to have 1*S*,2*S* absolute stereochemistry. X-ray structure determination of this product (which occurred as the hemihydrate in the solid state) confirmed the expected 1*S*,2*S* stereochemistry. A computer-generated perspective of the silylated diol 3 is shown in Figure 2, below. Although the absolute stereochemistry of 5 was certain (Flack χ absolute structure parameter = 0.1080 with esd = 0.0492), it was apparent that considerable disorder was present in the solid state of this compound, as evidenced by multiple. splitting occurrences for a number of the atoms present in the unit cell. This resulted in the rather poor standard crystallographic residual (R₁ of 0.1496) obtained for this structure determination. No attempt was made to further refine the molecules over the multiple occupancy sites, since our main objective of confirming the absolute stereochemistry had been met.

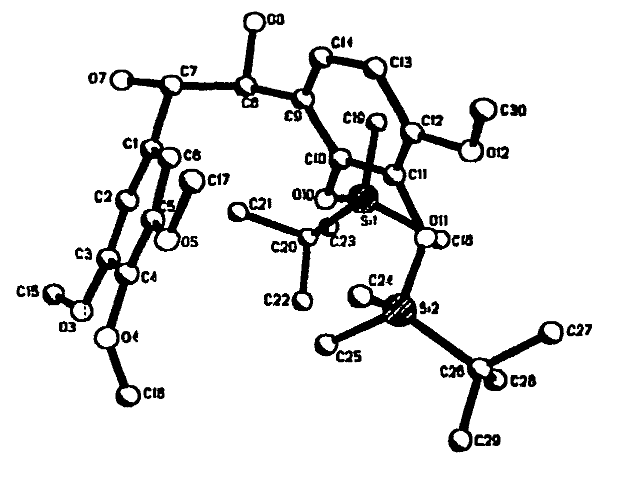

**Figure 2.** Crystal structure (excluding hydrogens) of (*S*,*S*)-diol (5).

Protected diol (*S*,*S*)-5 was next desilyated with tetrabutylammonium fluoride (TBAF) to obtain the tetraol (*S*,*S*)-diol **4b** in moderate yield (52%). Reaction of silyl ether (*S*,*S*)-diol **5** with acetic anhydride afforded (*S*,*S*)-diacetate **7**. Selective desilylation of (*S*,*S*)-diacetate **7** with TBAF proved to be difficult, yielding several products and suggesting concurrent oxidation. Other methods for desilylation (See: Sinhababu, A.K.; Kawase, M.; Borchardt, R.T. Synthesis **1988**, 710-711; Nelson, T.D.; Crouch, D. Synthesis **1996**, 1031-1069) were investigated, but none were able to displace TBAF as the desilyation agent of choice based on its efficient reaction time (*ca*. 20 min). The (*R*,*R*)-diols silyl ether 6, diacetate **8**, and tetraol 3b were prepared analogously using AD-mix-β. The relevant structures are illustrated in Scheme 1.

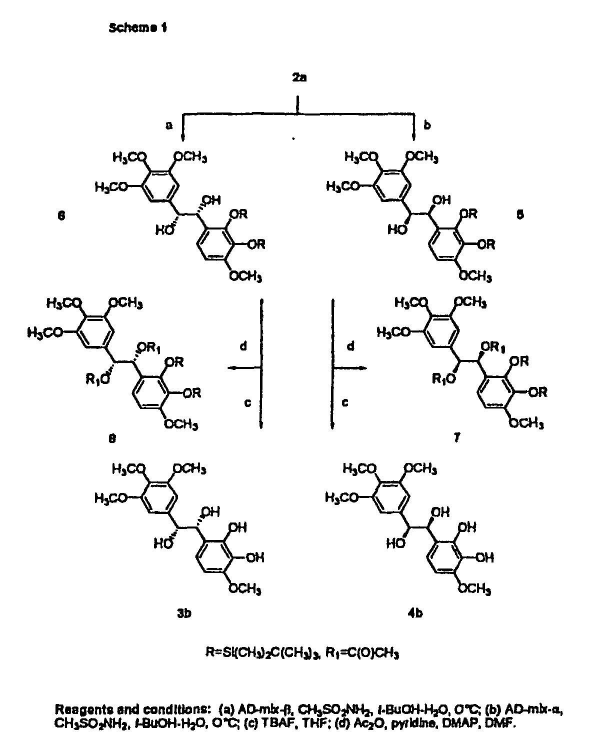

A comparison of the diols generated from (*E*)-olefin **2a** revealed a decrease in cancer cell growth inhibition from that of combretastatin A-1 **(1a)**, which is consistent with results obtained from the parallel set of diols prepared from the (*E*)-isomer of combretastatin A-4. Interesting to note is the fact that (*S*,*S*)-diol **4a,** a derivative of the *trans*-isomer of combretastatin A-4, is significantly more active than (*R*,*R*)-diol **3a** in the cancer cell lines employed (Table 1). In the combretastatin A-1 series, on the other hand, the difference noted in cancer cell line inhibition was negligible, and both (*S*,*S*)-diol **4b** and (*R*,*R*)-diol **3b** were substantially less active than (*S*,*S*)-diol **4a** from the A-4 series.

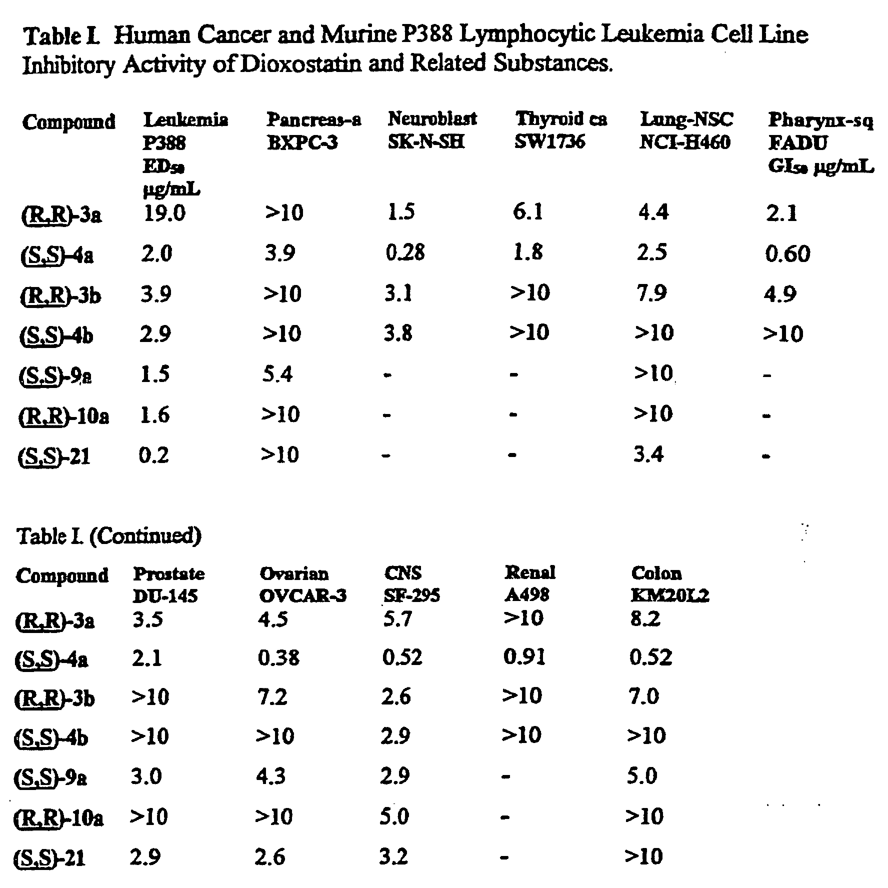

**Table II.**

| Inhibition of tubulin polymerization by diols 3-4 and combretastatin A-4 (1b) | | | |
|---|---|---|---|
| Compound | Inhibition of tubulin Polymerization ICso (µM) ± SD | Inhibition of colchicine binding Inhibitor: colchicine % inhibition ± SD | |
| **1a** | 1.1 ± 0.07 | 0.2:1 | 1:1 |
| | | 89 ± 3 | 99.6 ± 0.7 |
| | | 81 ± 4 | 98 ± 1 |
| **1b** | 1.0 ± 0.05 | | |
| (*R*,*R*)-**3a** | > 40 | | |
| (*S*,*S*)-**4a** | 14 ± 2 | | |
| (*R*,*R*)-**3b** | 20 ±2 | | |
| (*S*,*S*)-**4b** | 10 ±2 | | |
| **Footnote for Table II** In the polymerization experiments, the tubulin concentration was 10 µM. In the colchicine binding experiments, the tubulin concentration was 1.0 µM, and the [3H] colchicine concentration was 5.0 µM. See:Verdier-Pinard, P.; Lai, J-Y.; Yoo, H-D.; Yu, J.; Marquez, B.; Nagle, D.G.; Nambu, M.; White, J.D.; Falck, J.R.; Gerwick, W.H.; Day, B.W.; Hamel, E. Mol. Pharmacol. 1998, *53*, 62-76 for further details. All data with 1a were obtained with solutions prepared immediately before the experiments were performed. | | | |

The data presented in Table II, above, demonstrate that all four diols are much less potent than **1a** and **1b** as inhibitors of tubulin polymerization, in agreement with their much reduced cytotoxicity towards human cancer cell lines. The apparent preference of tubulin for (*S*,*S*)-diols **4a** and **4b** versus the analogous (*R*,*R*)-diols **3a** and **3b** is clear. The (*S*,*S*)-enantiomer is at least twice as inhibitory as the (*R*,*R*)-enantiomer. Specifically addressing the question of the importance of the second hydroxyl group in the combretastatin B-ring, the results with both the (*R*,*R*) pair and the (*S*,*S*) pair support the concept that the strength of the combretastatin interaction with tubulin is enhanced by the presence of the additional hydroxyl moiety. Curiously, the addition of this hydroxyl enhances the interaction with tubulin of the (*R*,*R*)-diol somewhat more (**3b** at least twice as active as **3a**) than of the (*S,S*)-diol (**4b** only 40% more active than **4a**). The greater difference between **3a** and **4a** than between **3b** and **4b** in their interactions with tubulin may also account for the greater difference between the former pair than the latter in the cytotoxicity results. However, relative affinities of the four diols for tubulin does not account for the observation that 4a is the most cytotoxic of the four compounds.

In addition, the data in Table II confirm the previously observed relative activities of **1a** and **1b**. **1a** appears to be more active as an inhibitor of colchicine binding.

Against microbe panels, the (*E*)-isomer of combretastatin A-1 was the most active compound, presumably due to its photoisomerization to the active *cis*-isomer (Table III). The difference in antimicrobial activity between parent compounds 1a and **1b** and their diol derivatives was unremarkable (Table III).

**Table III.**

| Antimicrobial activity of combretastatin A-1, A-4, B-1 and synthetic modifications | | |
|---|---|---|
| Compound | Microbe (s) initiated | Minimum inhibitory Concentration (µg/disk) |
| **1a** | *Staphylococcus aureus* | 50-100 |
| | *Neisseria gonorrhoeae* | 25-50 |
| **1b** | *N. gonorrhoeae* | 25-50 |
| | *N. gonorrhoeae* | 50-100 |
| | *N. gonorrhoeae* | 12.5-25 |
| **2b** | *S*. *aureus* | 50-100 |
| | *Streptococcus pneumoniae* | 50-100 |
| | *N. gonorrhoeae* | 6.25-12.5 |
| | *Stenotrophomonas maltophilia* | 50-100 |
| | *Candida albicans* | 50-100 |
| | *Cryptococcus neoformans* | 50-100 |
| **2d** | * | |
| (*R,R*)-**3a** | *S. pneumoniae* | 50-100 |
| (*S,S*)-**4a** | *S. pneumoniae* | 25-50 |
| (*R,R*)-**3b** | * | |
| (*S,S*)-**4b** | *N. gonorrhoeae* | 25-50 |

| | | |
|---|---|---|
| * at 100 µg/disk, no inhibition of the ten bacteria and fungi tested | | |

Further structural modifications aimed at converting the cancer cell line inactive (*E*)-isomers **2a** and **2c** into more active compounds was extended as follows. Diols **3b** and **4b**, synthesized from *trans*-stilbene **2a**, were further transformed by modification ofthe aliphatic hydroxyl groups in order to restrict free rotation. The methylenedioxy derivative (*cf*, **9** and **10**) was the rigid-ring of choice owing to its sterically small and relatively stable nature. While exploring the chemistry of stilbene **2a**, during previous evaluations of diphenol **1a** derivatives, the *tert*-butyldimethylsilyl (TBDMS) group became a liability owing to oxidative decomposition ofthe diphenol following desilylation with tetrabutylammonium fluoride (TBAF). Isolation of the resulting desilylated diphenols proved difficult and resulted in low yields. Therefore, the methoxymethyl (MOM) group was employed in a resynthesis of the corresponding (*E*)-isomer of **1a**, owing to its general stability (See: Yardley, J. P.; Fletcher, H. III. Introduction of the Methoxymethyl Ether Protecting Group. Synthesis 1975, 244.), and easy cleavage by dilute acid.

MOM protection of benzaldehyde **11** was accomplished in high yield with MOMCl. (See: Yardley, J. P.; Fletcher, H. III. Introduction of the Methoxymethyl Ether Protecting Group. Synthesis 1975, 244.). A Wadsworth-Emmons; (See: Baker, R.; Sims, R. J. A Convenient Modification of the Wadsworth-Emmons Method of Olefin Formation Using a Crown Ether. Synthesis 1981, 117.), reaction was used in preference to the conventional Wittig reaction to favor formation of the (*E*)-stilbene. Thus, phosphonate **14** was synthesized by reaction of benzyl bromide **13** with triethyl phosphite. Condensation of protected aldehyde **12** and phosphonate **14** in the presence of sodium hydride and 15-crown-5 afforded (E)-stilbene **15** in 70% yield. Asymmetric dihydroxylation of (*E*)-olefin **15** with AD-mix-α afforded the chiral diol (*S*,*S*)-**16**. The chirality of this diol was established by its relationship to the corresponding TBDMS derivative when the X-ray crystal structure determination was completed and was further supported by optical rotation data. (See: Figure 2). Synthesis of (*S*,*S*)-1,3-dioxolane **18** (See: Yardley, J. P.; Fletcher, H. III. Introduction of the Methoxymethyl Ether Protecting Group. Synthesis 1975, 244.), was conducted using 50% aqueous sodium hydroxide, dibromomethane and dichloromethane in the presence of a phase transfer catalyst. (See: Kim, K. S.; Sarek, W. A. Methylenation of Carbohydrates using Phase -Transfer Catalysis. Synthesis 1978, 48-50. Norman, D. G.; Reese, C. B.; Serafinowska, H. T. 2',3'-*O*-Methylene Derivatives of Ribonucleosides. Synthesis 1985, 751-754.). Removal of the MOM ether was accomplished by stirring **18** with 6N hydrochloric acid (aq) in 2-propanol-tetrahydrofuran at room temperature to afford (*S*,*S*)-1,3-dioxolane **9a,** later designated dioxostatin. The (*R,R*)-1,3-dioxolane **10a** was synthesized analogously **(15→19)** using AD-mix-β. The relevant structures are illustrated below in Scheme 2.

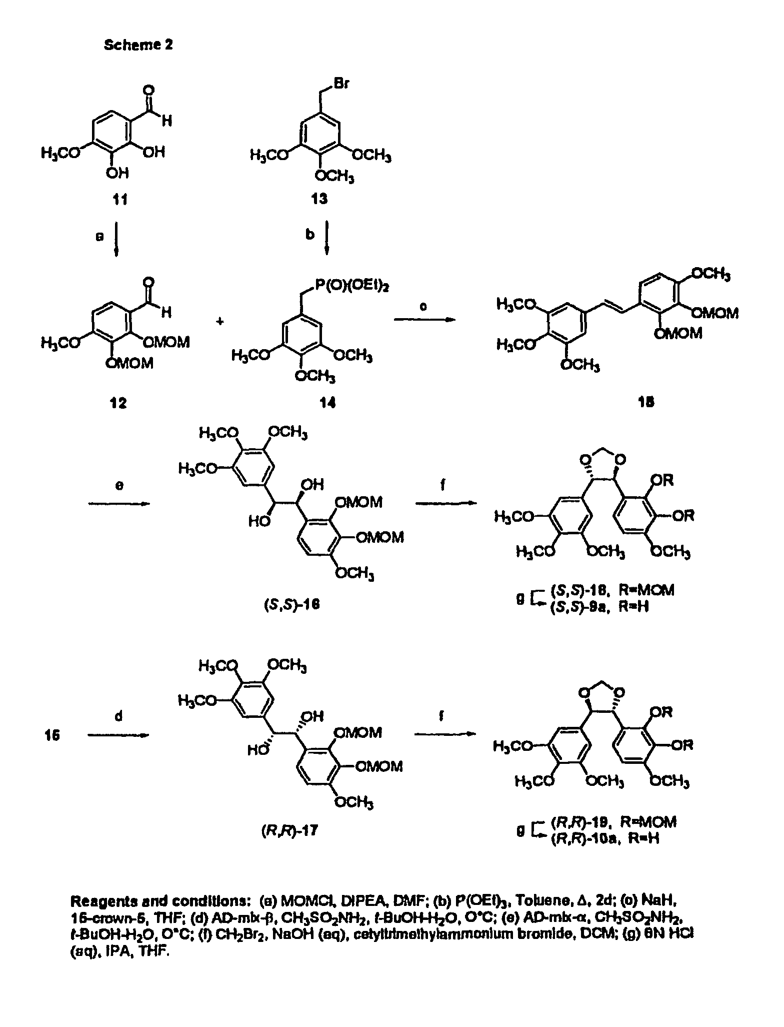

Biological evaluation of dioxolanes (*S*,*S*)-1,3-dioxolane **9a** and (*R*,*R*)-1,3-dioxolane **10a** (Table I) using the P388 lymphocytic leukemia cell line showed that they had similar levels of activity (ED₅₀ 1.5 µg/mL and ED₅₀ 1.6 µg/mL, respectively) in this system. However, the human tumor cell line data consistently showed (*S*,*S*)-1,3-dioxolane **9a** to be the more active, suggesting that the (*S*,*S*)-chirality present in dioxolane **9a** was more favorable than that in its enantiomer, **10a.** The biological potential of (*S*,*S*)-1,3-dioxolane **9a** was more fully appreciated when the inhibition of tubulin assembly was evaluated because (*S*,*S*)-1,3-dioxolane **9a** was 10-fold more potent than (*R*,*R*)-1,3-dioxolane 10a (0.59± 0.1 and 5.7± 0.8µM, respectively, Table IV). In fact, although less cytotoxic than combretastatin A-1 **(1a)** and A-4 **(1b),** 9a was twice as potent as the (Z)-stilbenes as an inhibitor of tubulin assembly. Its activity exceeded that of colchicine and was comparable to that ofthe potent antimitotic peptide dolastatin 10 (Table IV).

**Table IV.**

| Inhibition of Tubulin Assembly by Anticancer Drugs Acting on Areas Near the Colchicine or Vinca Sites^{a} | | | |
|---|---|---|---|
| Compound | Origin | IC₅₀ (µM±SD)^{a} | % Inhibition Colchicine binding ±SD (µM Inhibitor) |
| **1a** | Terrestrial Plant | 1.1±0.07 | 89±3 (1); 99.6±0.7 (5) |
| **1b** | Terrestrial Plant | 1.0±0.05 | 81±4 (1); 98±1 (5) |
| (*S,S*)-**9a** | Synthetic | 0.39±0.1 | 67±6 (1); 91±1 (5) |
| (*R*,*R*)-**10a** | Synthetic | 5.7±1 | 14±5 (5); 57±0.8 (50) |

| | | | |
|---|---|---|---|
| ^{a} SD, standard deviation. | | | |

Recently, dioxolanes **9b,c** and **10b,c** in the combretastatin series were synthesized and biologically evaluated by Shirai and co-workers. Initial investigations with these conformationally restricted combretastatin analogs dealt with the synthesis of 1,3-dioxolanes lacking a phenolic group on the aryl B-ring [(*S*,*S*)-**9b** and (*R*,*R*)-**10b**], which was earlier not considered essential for biological activity. The (*S*,*S*)-1,3-diaxolane **9b** was found to inhibit porcine brain tubulin polymerization with an IC₅₀ of 18 µM while (*R*,*R*)-1,3-dioxolane **10b** was inactive exhibiting an ICso of >100 µM. Eventually, Shirai and co-workers evaluated the series of 1,3-dioxolanes in which the 3-position on the B aryl ring contained the phenolic group [(*S*,*S*)-**9c** and (*R*,*R*)-**10c**]. They found that (*S*,*S*)-1,3-dioxolane **9c** showed even greater potency than did (*S*,*S*)-1,3-dioxolane **9b** with an IC₅₀ of 4-6 µM, while (*R*,*R*)-1,3-dioxolane **10c** was again found to be inactive exhibiting an IC₅₀ of >50 µM. These data indicated that the (*S*,*S*)-1,3-dioxolane group present in **9b** and **9c** assumes a favorable conformation for tubulin binding while the (*R*,*R*)-1,3-dioxolanes **10b** and **10c** do not. Furthermore, the most active constituent, (*S*,*S*)-1,3-dioxolane **9c** derived from combretastatin A-4, inhibited the *in vitro* growth of PC-6 and PC-12 (multidrug resistant) cancer cell lines at a GI₅₀ of 0.002 µg/mL.

The greater potency exhibited by dioxostatin **(9a)** when compared to both (*S*,*S*)-1,3-dioxolanes **9b** and **9c** indicates that the vicinal hydroxyls at the 2-and 3-position of the B ring are essential for potent inhibition of tubulin polymerization. The apparently greater activity of **9a** relative to **9c** with tubulin seems to magnify the small but reproducible differences generally observed when combretastatin A-1 and combretastatin A-4 are compared (Table IV). (In most direct comparisons, the two (Z)-stilbenes have comparable effects on tubulin assembly, but combretastatin A-1 is slightly more potent as an inhibitor of colchicine binding). Also of interest is the fact that (*R*,*R*)-1,3-dioxolane **10a** exhibits activity nominally similar to that of (*S*,*S*)-1,3-dioxolane **9c** (IC₅₀ of 5.7 µM and IC₅₀ of 4-6 µM respectively, however, different reaction conditions and protein preparations were used), which further supports the conclusion that the vicinal hydroxyl unit at the 2-and 3-positions of the B-ring enhance the interaction of these compounds with tubulin.

The microtubule system of eukaryotic cells is an important target for the development of anticancer agents. Since dioxostatin (9a) represents a major advance in inhibition of tubulin assembly it became a target for preclinical development. Toward that objective, the synthesis of (*S,S*)-1,3-dioxolane prodrug **21** was initiated. Diphosphorylation (See: Pettit, G.R.; Rhodes, M.R. Antineoplastic Agents 389). New Synthesis of Combretastatin A-4 Prodrug. Anti-Cancer Drug Des. 1998, **13**, 183-191; Pettit, G.R.; and Lippert, J.W. III. Antineoplastic Agents 429. Synthesis of Combretastatin A-1 and Combretastatin B-1 Prodrugs. Anti-Cancer Drug Des. 2000, (**accepted**); and Silverberg, L. J.; Dillon, J. L.; and Vemisheti, P. A Simple, Rapid and Efficient Protocol for the Selective Phosphorylation of Phenols with Dibenzyl Phosphite. *Tetrahedron Lett*. **1996**, *37*, 771-774), of dioxostatin **(9a)** was performed with dibenzyl phosphite to create diphosphate 20. Hydrogenolysis of the benzyl groups under a slight positive pressure of hydrogen followed by reaction of the diphosphoric acid with sodium methoxide in anhydrous methanol afforded the tetrasodium phosphate prodrug (*S*,*S*)-**21** in good yield. The diphosphate derivative was chosen owing to its biolability by phosphatases and favorable water solubility. Phosphate prodrugs have proven to be valuable improvements when deployed with certain anticancer and other types of drugs such as combretastatins A-1 and A-4, pancratistatin, tyrosine-containing peptides and etoposide. The relevant structures are illustrated below in Scheme 3. 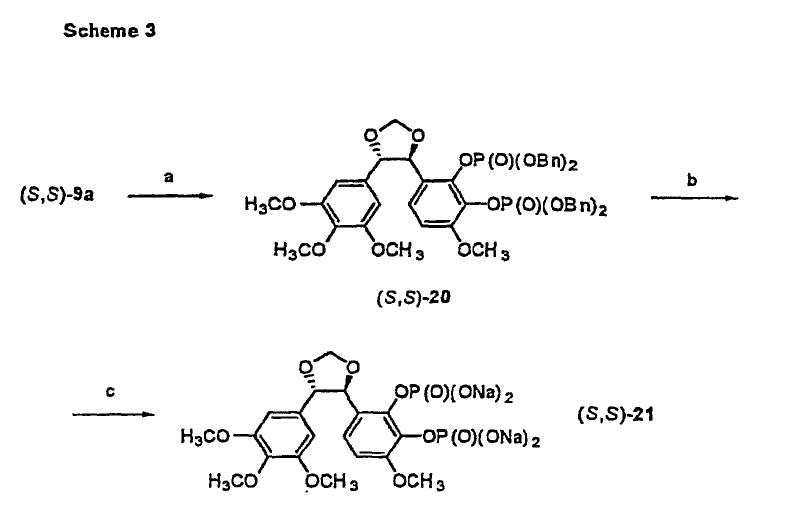 **Reagents and conditions:** (a) HP(O)(OBn)₂, DMAP, DIPEA, CCl₄, CH₃CN,-10°C; (b) H₂, 10% Pd/C, EtOH; (c) NaOCH₃, anhydrous MeOH.

The (*S*,*S*)-1,3-dioxolane prodrug **(21)** was found to exhibit an EDso of 0.2 µg/mL against the murine P388 lymphocytic leukemia cell line, an approximate 10-fold increase in activity over the parent compound, dioxostatin **9a**. Also, diphosphate **21** was shown to have an aqueous solubility of >35 mg/mL. Prodrug **21** was inactive as an inhibitor of tubulin assembly, and has been the case with all other phosphorylated derivatives in this family of compounds.

### Experimental Section

**General Experimental Procedures.** Ethyl acetate (EtOAc), hexane, and dichloromethane (DCM) were redistilled. Diisopropylethylamine (DIPEA) 4-dimethylaminopyridine (DMAP), *tert*-butyl alcohol, and dibromomethane were from the Fisher-Acros Chemical Company, and all other reagents including methoxymethyl chloride (MOMCl) were from the Sigma-Aldrich Chemical Company. Solvent extracts of aqueous solutions were dried over anhydrous sodium sulfate. Column chromatography was performed using either flash silica gel (230-400 mesh ASTM) or gravity silica gel (70-230 mesh ASTM). Analtech silica gel GHLF plates were used for TLC, and compounds were visible with fluorescent short-wave light (254 nm).

Melting points were determined using an Electrothermal 9100 apparatus and are uncorrected. The ¹H NMR spectra were prepared employing a Varian VXR-300 MHz or Unity 500 MHz instrument and referenced to TMS as the internal standard. Mass spectral data were recorded using a Varian MAT 312 instrument (EIMS), or Vestec Lasertec Research mass spectrometer incorporating a Laser Sciences nitrogen laser (337 nm light pulses of 3 ns duration with 4-hydroxybenzylidenemalononitrle as the matrix and cytochrome c as the external standard for calibration purposes, (TOFMS). (See: Brune, D.C.; Cheung, J. 4-Hydroxybenzylidenemalononitrile: A Useful matrix for MALDI of Water-Insoluble Analytes. Proc. 45^{th} ASMS Conference on Mass Spectrometry and Allied Topics, Palm Springs, California, June 1-5, 1997). The IR spectra were obtained with a Mattonson Instruments 2020 Galaxy Series FTIR instrument. UV spectra were obtained employing a Hitachi U-2000 UV/VIS spectrophotometer. Optical rotation values were recorded employing a Perkin Elmer 241 polarimeter. Elemental analyses were determined by Galbraith Laboratories, Inc., Knoxville, TN.
**(1*R*,2*R*)-1,2-Dihydroxy-1-[2',3'-(di(*tert*-butyldimethylsilyloxy)-4'-methoxyphenyl]-2-(3",4",5"-trimethoxyphenyl)-ethane (6).** To a 100-mL foil-wrapped round bottom flask, containing a magnetic stir bar, was added distilled water (14 mL), *tert*-butyl alcohol (14 mL), AD-mix-β (3.8 g; 1.40 g per mmol), and methanesulfonamide (0.27 g; 2.85 mmol). Before being cooled to 0 °C, the mixture was stirred at room temperature for several min. Some of the compounds began to precipitate and 2',3'-bis(*tert*-butyldimethylsilyloxy)-3,4,4',5-tetramethoxy-(*E*)-stilbene **(2a)** (See:Pettit, G.R.; Lippert, III, J.W.; Pettit, R.K. Anticancer Drug Design **in** preparation) (1.4 g; 2.54 mmol) was added at once, and the slurry was stirred vigorously at 0°C for 8 hours and at room temperature for 24 hours. Sodium sulfite (3.9 g; 30.9 mmol; 12 eq) was added and the mixture stirred for an additional 0.5 hours. The product was extracted with EtOAc (4 x 50 mL), and the combined organic phase was washed with 2*N* KOH (aq) and dried. Evaporation of the solvent *in vacuo* produced an off-yellow solid that was absorbed onto silica gel and subjected to flash column chromatography (eluent gradient: 9:1 to 1:1 hexane-EtOAc) to afford a clear oil, which crystallized from hexane as a colorless fluffy solid (0.94 g; 74% based on 0.23 g of **2a** recovered): m.p. 122-123 °C; R_{f} 0.50 (1:1, hexane-EtOAc); [α]_{D}²⁵ +46° (*c* 1.2, CHCl₃); IR (nujol) vₘₐₓ 3526, 1595, 1251, 1124, 1097, 831, 781 cm⁻¹; ¹H NMR (300 MHz, CDCl₃) δ -0.16 (3H, s, SiCH₃), -0.050 (3H, s, SiCH₃), 0.040 (3H, s, SiCH₃), 0.17 (3H, s, SiCH₃), 0.89 (9H, s, C₄H₉), 1.01 (9H, s, C₄H₉), 2.45 (1H, d, *J* = 1.7 Hz, OH, D₂O exchanged), 2.89 (1H, d, *J* = 1.2 Hz, OH, D₂O exchanged), 3.72 (6H, s, 2 x OCH₃), 3.75 (3H, s, OCH₃), 3.76 (3H, s, OCH₃), 4.66 (1H, dd, *J* = 7.5 Hz, *J* = 2.4 Hz, CH), 5.06 (1H, dd, *J* = 7.8 Hz, *J* = 3.3 Hz, CH), 6.33 (2H, s, H-2", H-6"), 6.59 (1H, d, *J* = 8.1 Hz, H-5'), 7.05 (1H, d, *J* = 8.7 Hz, H-6'); EIMS *m*/*z* 594 (M⁺, 5), 519 (95), 397 (100), 339 (10), 198 (95); *anal*. C 59.70%, H 8.50%, calcd for C₃₀H₅₀O₈Si₂•1/2H₂O, C 59.80%, H 8.50%.
**(1*R*,2*R*)-1,2-Dihydroxy-1-(2',3'-dihydroxy-4'-methoxyphenyl)-2-(3",4",5"-trimethoxyphenyl)-ethane (3b).**
To a solution of (*R,R*)-diol **6** (0.24 g; 0.397 mmol) in anhydrous THF (4 mL), was added TBAF (0.84 µL; 0.84 mmol; 2.1 eq; 1 M in THF soln) in a previously flame-dried flask After 20 min the reaction was terminated with ice-cold 6N HCl and extracted with EtOAc (4 x 20 mL). The combined organic phase was washed with saturated NaCl (aq) and dried. Removal of the organic solvent yielded a dark oil, which was subjected to column chromatography (66:33:1 EtOAc-hexane-AcOH) to afford a light brown oil that crystallized from EtOAc-hexane as a colorless solid (75 mg; 52%): m.p. 64-66 °C; R_{f} 0.36 (66:33:1, hexane-EtOAc-AcOH); [α]_{D}²⁵+56° (*c* 1.1, CHCl₃); IR (film) vₘₐₓ 3425, 2939, 1593, 1454, 1327, 1234, 1145 cm⁻¹; ¹H NMR (300 MHz, CDCl₃) δ 3.70 (6H, s, 2 x OCH₃), 3.79 (3H, s, OCH₃), 3.80 (3H, s, OCH₃), 4.67 (1H, d, *J* = 7.8 Hz, CH), 4.83 (1H, d, *J* = 7.2 Hz, CH), 6.12 (1H, d, *J* = 8.7 Hz, H-5'), 6.24 (1H, d, *J* = 9.0 Hz, H-6'), 6.33 (2H, s, H-2", H-6"); EIMS *m*/*z* 348 (M⁺-H₂O, 60), 319 (95), 196 (100), 167 (10), 153 (25); *anal*. C 57.59%, H 6.18%, calcd for C₁₈H₂₂O₈•1/2H₂O, C 57.41%, H 6.25%.
(1*S*,2*S*)-1,2-Dihydroxy-1-[2',3'-di(*tert*-butyldimethylsilyloxy)-4'-methoxyphenyl]-2-(3",4",5"-trimethoxyphenyl)-ethane (5). The asymmetric dihydroxylation reaction conditions described above for preparation of (*R*,*R*)-diol **6** were applied to (*E*)-olefin **2a** (1.56 g; 2.78 mmol) using AD-mix-α to afford (*S*,*S*)-diol **5** as a colorless fluffy solid (1.2 g; 75%): m.p. 122-123 °C; R_{f} 0.50 (1:1, hexane-EtOAc); [α]_{D}²⁵ -46° (*c* 1.3, CHCl₃); IR (nujol) vₘₐₓ 3524, 2361, 1593, 1251, 1124, 1097, 954, 831 cm⁻¹; ¹H NMR (300 MHz, CDCl₃) δ -0.16 (3H, s, SiCH₃), -0.05 (3H, s, SiCH₃), 0.04 (3H, s, SiCH₃), 0.17 (3H, s, SiCH₃), 0.89 (9H, s, C₄H₉), 1.01 (9H, s, C₄H₉), 2.44 (1H, d, *J* = 3.3 Hz, OH, D₂O exchanged), 2.87 (1H, d, *J* =1.2 Hz, OH, D₂O exchanged), 3.72 (6H, s, 2 x OCH₃), 3.75 (3H, s, OCH₃), 3.76 (3H, s, OCH₃), 4.66 (1H, dd, *J* = 7.8 Hz, *J*= 2.1 Hz, CH), 5.06 (1H, dd, *J*= 7.5 Hz, *J*=3.3 Hz, CH), 6.33 (2H, s, H-2", H-6"), 6.59 (1H, d, *J* = 8.7 Hz, H-5'), 7.06 (1H, d, *J* = 8.4 Hz, H-6'); EIMS *m*/*z* 594 (M⁺, 10), 519 (95), 397 (100), 339 (20), 198 (100); *anal*. C 59.70%, H 8.50%, calcd for C₃₀H₅₀O₈Si₂•1/2H₂O, C 59.66%, H 8.61%.
X-Ray Crystal Structure of (1*S*,2*S*)-1,2-dihydroxy-1-[2',3'-bis-(*tert*-butyl-dimethyl-silanyloxy)-4'-methoxy-phenyl]-2-(3",4",5"-trimethoxy-phenyl)-ethane hemihydrate (5). A thin plate, 0.40 x 0.40 x 0.06 mm, obtained from a methanol-hexane solution, was mounted on the tip of a glass fiber with Super Glue. Data collection was performed at 27°C for a monoclinic system, with all reflections corresponding to slightly more than a complete quadrant (2θ < 130°) being measured using an ω/2θ scan technique. Friedel reflections were also collected, whenever possible, immediately after each original reflection. Subsequent statistical analysis of the complete reflection data set using the XPREP (See: *SHELXTL-PC, Version 5.1*, 1997, an integrated soft-are system for the determination of crystal structures from diffraction data, Bruker Analytical X-ray Systems, Inc., Madison, WI 53719. This package includes, among others: XPREP, an automatic space group determination program; XS, the Bruker SHELXS module for the solution of X-ray crystal structures from diffraction data; XL, the Bruker SHELXL module for structure refinement; XP, the Bruker interactive graphics display module for crystal structures) program indicated the space group was P2₁. Each asymmetric unit of the cell was found to contain four independent molecules of the parent molecule, as well as two molecules of water. Crystal data: C₃₀H₅₀O₈ • 1/2 H₂O, a = 13.303(2), b = 32.475(7), c = 19.960(4) Å, V = 7179(3) Å³, λ (Cu K_{α}) = 1.54178 Å, ρ_{c} = 1.117 g cm⁻³ for Z = 8 and F.W. = 603.89, F(000) = 2616. After Lorentz and polarization corrections, merging of equivalent reflections and rejection of systematic absences, 19017 unique reflections (R(int) =0.1195) remained, of which 13433 were considered observed (Iₒ > 2σ(Iₒ)) and were used in the subsequent structure solution and refinement. Linear and anisotropic decay corrections were applied to the intensity data as well as an empirical absorption correction (based on a series of psi-scans), (See: Lin, C.M.; Singh, S.B.; chu, P.S.; Dempcy, R.O.; Schmidt, J.M.; Pettit, G.R.; Hamel, E. Interactions of Tubulin with Potent Natural and Synthetic Analogs of the Antimitotic Agent Combretastatin: A Structure-Activity Study. Mol Pharmacol. **1998,** *34*, 200-208; Pettit, G.R.; Singh, S.B.; Schmidt, J.M. Isolation, Structure and Synthesis of Combretastatins B-3 and B-4 from *Combretum caffrum*. J. Nat. Prod. **1988,** *51*, 517-527; Nandy, P.; Samitendu, B.; Gao, H.; Hui, M.B.V.; Lien, E.J. Quantitative Structure-Activity Relationship Analysis of combretastatins: A Class of Novel antimitotic agents. Pharm. Res. **1991,** *8*, 776-781; Roberson, R.W.; Tucker, B.; Pettit, G.R. Microtubule depolymerization in *Uromyces appendiculatus* by Three New Antineoplastic Drugs: Combretastatin A-4, Dolastatin 10, and Halichendrin B. Mycol. Res. **1998,** *102*, 378-382; Iwasakik, S. Antimitotic Agents: Chemistry and Recognition of Tubulin Molecule. Medicinal Research Rev. **1993,** *13*, 183-198). Structure determination was accomplished with SHELXS (See: *SHELXTL-PC, Version 5.1*, **1997,** an integrated software system for the determination of crystal structures from diffraction data, Bruker Analytical X-ray Systems, Inc., Madison, WI 53719. This package includes, among others: XPREP, an automatic space group determination program; XS, the Broker SHELXS module for the solution of X-ray crystal structures from diffraction data; XL, the Bruker SHELXL module for structure refinement; XP, the Bruker interactive graphics display module for crystal structures). All non-hydrogen atoms for **5**, including the water solvate atoms, were located using the default settings of that program. The remaining hydrogen atom positions were calculated at optimum positions. The latter atoms were assigned thermal parameters equal to 1.2 or 1.5 (depending upon structural atom type) the Uiso value of the atom to which they were attached and then both coordinates and thermal values were forced to ride that atom during final cycles of refinement. All non-hydrogen atoms were refined anisotropically in a full-matrix least-squares refinement process with SHELXL (See: *SHELXTL-PC, Version 5.1*, **1997**, an integrated software system for the determination of crystal structures from diffraction data, Bruker Analytical X-ray Systems, Inc., Madison, WI 53719. This package includes, among others: XPREP, an automatic space group determination program; XS, the Bruker SHELXS module for the solution of X-ray crystal structures from diffraction data; XL, the Bruker SHELXL module for structure refinement; XP, the Bruker interactive graphics display module for crystal structures) in the SHELXTL-PC software package. The final standard residual **R** value for the model shown in Figure 1 was 0.1484 for observed data (13433 reflections) and 0.1850 for all data (19017 reflections). The corresponding Sheldrick **R** values were wR₂ of 0.3706 and 0.4031, respectively. The Flack absolute structure parameter χ was 0.05 (5) for the model depicted in Figure 1, thus indicating that the absolute stereochemistry shown (and expected) for **5** is correct, i.e., 1*S*, 2*S*. A final difference Fourier map showed residual electron density attributed solely to the silicon atoms; the largest difference peak and hole being 0.61 and -0.55 e/Å³, respectively. Final bond distances and angles were all within acceptable limits.
**(1*S*,2*S*)-1,2-Dihydroxy-1-(2',3'-dihydroxy-4'-methoxyphenyl)-2-(3",4",5"-trimethoxyphenyl)-ethane (4b).** Silyl ether (*S*,*S*)-diol **5** (0.36 g; 0.610 mmol) was desilylated as described above for the synthesis of (*R*,*R*)-diol **3b** and afforded (*S*,*S*)-diol **4b** as a colorless solid (0.13 g; 59%): m.p. 64-66 °C; R_{f} 0.36 (66:33:1 hexane-EtOAc-AcOH); [α]_{D}²⁵ -52° (c 0.99, CHCl₃); IR (film) vₘₐₓ 3418, 2939, 1593, 1510, 1462, 1327, 1290, 1234, 1124 cm⁻¹; ¹H NMR (300 MHz, CDCl₃) δ 3.73 (6H, s, 2 x OCH₃), 3.81 (3H, s, OCH₃), 3.83 (3H, s, OCH₃), 4.70 (1H, d, *J* = 7.2 Hz, CH), 4.88 (1H, d, *J* = 7.2 Hz, CH), 6.15 (1H, d, *J* = 8.1 Hz, H-5'), 6.27 (1H, d, *J*= 8.1 Hz, H-6'), 6.37 (2H, s, H-2", H-6"); EIMS *m*/*z* 348 (M⁺-H₂O, 20), 319 (15), 196 (100), 168 (20), 153 (10); *anal*. C 57.59%, H 6.18%, calcd for C₁₈H₂₂O₈•1/2H₂O, C 57.61%, H 6.28%.
**(1*R*,2*R*)-1,2-Diacetoxy-1-[2',3'-di-(*tert*-butyldimethylsilyloxy)-4'-methoxyphenyl]-2-(3",4",5"-trimethoxyphenyl)-ethane (8).** To a solution of (*R,R*)-diol **6** (0.21 g; 0.351 mmol) in anhydrous DCM (2 mL) was added acetic anhydride (26 mL; 2.7 mmol; 7.8 eq), pyridine (0.20 mL; 2.35 mmol; 6.7 eq), and a catalytic amount of DMAP (5 mg). After 3 hours, the reaction was terminated with ice-water and extracted with EtOAc (4 x 25 mL). The combined organic phase was washed with 2N HCl followed by 10% NaHCO₃ (aq) and dried. Removal (reduced pressure) of solvent yielded a clear oil that was separated by flash column chromatography (1:1 hexane-EtOAc) to afford a crude product, which crystallized from hexane as a colorless solid (0.24 g; 99%): m.p. 128-129 °C; R_{f} 0.63 (2:1, hexane-EtOAc); [α]_{D}²⁵ -22° (*c* 1.4, CHCl₃); EIMS *m*/*z* 678 (M⁺, 5), 621 (40), 439 (60), 397 (100), 73 (30); *anal*. C 60.14%, H 8.01%, calcd for C₃₄H₅₄O₁₀Si₂, C 60.38%, H 8.09%.
**(1*S*,2*S*)-1,2-Diacetyoxy-[2',3'-di-(*tert*-butyldimethylsilyloxy)-4'-methoxyphenyl]-2-(3",4",5"-trimethoxyphenyl)-ethane (7).** The silyl ether (*S*,*S*)*-*diol **5** (0.25 g; 0.42 mmol) was acylated as described above for the synthesis of (*R*,*R*)-diacetate **8** to afford a colorless solid (0.28 g; 98%): m.p. 128-129 °C; R_{f} 0.63 (2:1, hexane-EtOAc); [α]_{D}²⁵ +20° (*c* 1.3, CHCl₃); TOFMS *m*/*z* 717,(M+K)⁺; *anal*. C 60.14%, H 8.01%, calcd for C₃₄H₅₄O₁₀Si₂, C 60.08%, H 8.08%.
**4-Methoxy-2,3-di(methoxymethoxy)-benzaldehyde (12).** DIPEA (7.9 mL; 45 mmol; 3 eq) was added *via* syringe to a solution of 2,3-dihydroxy-4-methoxy-benzaldehyde **(11)** (2.5 g; 15 mmol) in DMF (20 mL) at 0 °C. After 5 minutes, MOMCl (2.6 g; 33 mmol; 2.5 eq) was added over 10 min and stirring was continued for 1 hour at 0 °C. The reaction was terminated with ice-water. The mixture was then extracted with EtOAc (4 x 50 mL), and the organic phase washed with 10% NaHCO₃ (aq) and dried. Evaporation of the solvent (reduced pressure) yielded a brown oil, which was absorbed onto silica gel and subjected to flash column chromatography (5:1 hexane-EtOAc) to afford a clear oil, 3.5 g (90%): b.p. 164-165 °C/(0.11 mm Hg); R_{f} 0.51 (3:1, hexane-EtOAc); EIMS *m*/*z* 256 (M⁺, 5%), 211 (10%), 180 (15%), 151 (10%), 46 (100%); IR (film) vₘₐₓ 2945, 1689, 1587, 1494, 1454, 1383, 1284, 1159, 1080, 808 cm⁻¹; ¹H NMR (300 MHz, CDCl₃) δ 3.57
(3H, s, OCH₂OCH₃), 3.61 (3H, s, OCH₂OCH₃), 3.93 (3H, s, OCH₃) 5.13 (2H, s, OCH₂OCH₃), 5.27 (2H,s, OCH₂OCH₃), 6.81 (1H, d, *J =* 8.7 Hz, H-5), 7.66 (1H, d, *J* = 8.7 Hz, H-6), 10.29 (1H, s, CHO). Anal. Calcd. For C₁₂H₁₆O₆: C, 56.28%: H, 6.46%. Found: C, 56.29%: H, 6.42%.
**Diethyl 3,4,5-trimethoxybenzylphosphonate (14)**. A solution of triethyl phosphite (28 mL; 163 mmol; 1 eq) in toluene (100 mL) was added to a stirred solution of 3,4,5-trimethoxybenzyl bromide (13, 43 g; 162 mmol) in toluene (100 mL), and the mixture was heated at reflex for 18 hours and stirred at RT for 6 hours. Evaporation (*in vacuo*) of the solvent resulted in a crude oil, which was distilled (34 g; 66%): b.p. 212-214 °C/(water aspirator); R_{f} 0.14 (2:1, EtOAc-hexane); EIMS *m*/*z* 318 (M⁺, 100%), 303 (25%), 195 (10%), 181 (95%), 167 (20%); IR (film) vₘₐₓ 3462, 2982, 1591, 1504, 1454, 1334, 1244, 1124, 1026, 964 cm⁻¹; ¹H NMR (300 MHz, CDCl₃) δ 1.27 (6H, t, *J =* 7.2 Hz, 2 x OCH₂CH₃), 3.09 (2H, d, *J =* 21 Hz, Ph-CH₂), 3.83 (3H, s, OCH₃), 3.85 (6H, s, 2 x OCH₃), 4.05 (4H, q, *J* = 7.2 Hz, 2 x OCH₂CH₃), 6.53 (2H, d, J= 3.0 Hz, H-2, H-6). Anal. Calcd. For C₁₄H₂₃O₆P•¼ H₂O: C, 52.09%: H, 7.33%. Found: C, 52.30%: H, 7.32%.
**3,4,4',5-Tetramethoxy-2',3'-di(methoxymethoxy)-(*E*)-stilbene (15).** A solution of protected aldehyde **12** (9.3 g; 36.3 mmol; 1 eq) and phosphonate **14** (12 g; 38.0 mmol; 1 eq) in anhydrous THF (50 mL) was added to a stirred slurry of NaH (1.5 g; 38.0 mmol, 60% dispersion in mineral oil; 1 eq), and 15-crown-5 (0.22 mL; 1.11 mmol; 0.03 eq) in anhydrous THF (50 mL) at 0 °C. After 12 hours of stirring, the reaction was stopped with H₂O. The reaction mixture was then extracted with EtOAc (4 x 100 mL), and the organic phase washed with sat. NaCl (aq) and 10% NaHSO₃ (aq) and dried. Removal of the solvent under reduced pressure yielded a yellow oil, which was subjected to flash column chromatography (5:1 hexane-EtOAc) to afford a clear oil that crystallized from EtOAc-hexane (11 g; 70%): m.p. 96-97 °C; R_{f} 0.38 (3:1, hexane-EtOAc); EIMS *m*/*z* 420 (M⁺, 100%), 375 (50%), 344 (100%), 329 (100%), 181 (40%); IR (film) vₘₐₓ 2941, 1581, 1504, 1464, 1240, 1126, 1010, 970, 927, 821 cm⁻¹; ¹H NMR (300 MHz, CDCl₃) δ 3.56 (3H, s, OCH₂OCH₃), 3.62 (3H, s, OCH₂OCH₃), 3.87 (3H, s, OCH₃), 3.88 (3H, s, OCH₃), 3.90 (6H, s, 2 x OCH₃), 5.14 (2H, s, OCH₂OCH₃), 5.18 (2H, s, OCH₂OCH₃), 6.74 (2H, s, H-2, H-6), 6.74 (1H, d, *J* = 9.0 Hz, H-5'), 6.93 (1H, d, *J* = 16 Hz, -CH=CH-), 7.34 (1H, d, *J* = 16 Hz, -CH=CH-), 7.34 (1H, d, *J =* 8.7 Hz, H-6'). Anal. Calcd. For C₂₂H₂₈O₈: C, 62.85%: H, 6.71%. Found: C, 62.95%: H, 6.71%.
**(1*S*,2*S*)-1,2-Dihydroxy-1-[4'-methoxy-2',3'-di(methoxymethoxy)-phenyl]-2-(3",4",5"-trimethoxyphenyl)-ethane (16).** The asymmetric dihydroxylation reaction conditions described below for obtaining (*R,R*)-diol **17** were applied to (*E*)-olefin **15** (1.4 g; 3.3 mmol) using AD-mix-α to afford diol (*S*,*S*)-diol 16 as a colorless fluffy light solid that recrystallized from hexane (1.3 g; 82%): m.p. 122-123 °C; R_{f} 0.37 (2:1, hexane-EtOAc); [α]_{D}²⁵ -83° (c = 0.79, CHCl₃); EIMS *m*/*z* 436 (M⁺ -H₂O, 5%), 331 (10%), 257 (10%), 197 (20%), 181 (60%); IR (film) vₘₐₓ 3454, 2941, 1593, 1498, 1456, 1236, 1157, 970, 927 cm⁻¹; ¹H NMR (300 MHz, CDCl₃) δ 3.53 (3H, s, OCH₂OCH₃), 3.56 (3H, s, OCH₂OCH₃), 3.73 (6H, s, 2 x OCH₃), 3.78 (3H, s, OCH₃), 3.81 (3H, s, OCH₃), 4.66 (1H, d, *J* = 7.2 Hz, OCH₂OCH₃), 4.98 (1H, dd, *J =* 6.0 Hz, *J =* 2.0 Hz, OCH₂OCH₃), 5.06 (2H, s, OCH₂OCH₃), 5.09 (1H, *d, J =* 5.7 Hz, CH), 5.21 (1H, d, *J* = 5.4 Hz, CH), 6.42 (2H, s, H-2", H-6"), 6.59 (1H, d, *J* = 8.7 Hz, H-5'), 6.80 (1H, d, *J* = 8.7 Hz, H-6'). Anal. Calcd. For C₂₂H₃₀O₁₀: C, 58.14%: H, 6.65%. Found: C, 58.24%: H, 6.65%.
**(1*R*,2*R*)-1,2-Dihydroxy-1-[4'-methoxy-2',3'-di(methoxymethoxy)phenyl]-2-(3",4",5"-trimethorypheayl)-ethane (17).** To a 50 mL foil wrapped round bottom flask, equipped with a magnetic stirrer, was added distilled water (20 mL), *tert*-butyl alcohol (20 mL), AD-mix-β (5 g; 1.40 g per mmol), and methanesulfonamide (0.34 g; 2.9 mmol). The mixture was stirred at room temperature for several min and cooled to 0 °C, whereupon some of the salts precipitated. Stilbene **15** (1.4 g; 3.3 mmol) was added at once, and the slurry stirred vigorously at 0 °C for 8 hours and room temperature for 24 hours. Sodium sulfite (3.9 g; 31 mmol; 12 eq) was added and the mixture was stirred for an additional 0.5 hours. The mixture was extracted with EtOAc (4 x 25 mL), and the combined organic extract washed with 2N KOH (aq) and dried. Evaporation *in vacuo* of the solvent yielded a straw-colored solid, that was subjected to flash column chromatography (eluent gradient: 3:1 to 1:4 hexane-EtOAc) to afford (*R,R*)-diol **17** as a colorless and fluffy solid which was recrystallized from hexane (1.4 g; 89%): m.p. 122-123 °C; R_{f} 0.37 (2:1, hexane-EtOAc); [α]_{D}²⁵ +85° (c = 0.87, CHCl₃); EIMS *m*/*z* 454 (M⁺, 5%), 257 (20%), 242 (20%), 198 (40%), 181 (85%); IR (film) vₘₐₓ 3445, 2941, 1593, 1498, 1456, 1236, 1157, 1126, 972, 927 cm⁻¹; ¹H NMR (300 MHz, CDCl₃) δ 3.53 (3H, s, OCH₂OCH₃), 3.55 (3H, s, OCH₂OCH₃), 3.73 (6H, s, 2 x OCH₃), 3.78 (3H, s, OCH₃), 3.82 (3H, s, OCH₃), 4.66 (1H, d, *J =* 7.2 Hz, OCH₂OCH₃), 4.97 (1H, dd, *J* = 7.5 Hz, *J* = 2.1 Hz, OCH₂OCH₃), 5.06 (2H, s, OCH₂OCH₃), 5.08 (1H, *d, J =* 5.4 Hz, CH), 5.21 (1H, d, *J* = 6.3 Hz, CH), 6.42 (2H, s, H-2", H-6"), 6.59 (1H, d, *J* = 9.0 Hz, H-5'), 6.80 (1H, d, *J* = 8.7 Hz, H-6'). Anal. Calcd. For C₂₂H₃₀O₁₀: C, 58.14%: H, 6.65%. Found: C, 58.17%: H, 6.79%. (4*S*,5*S*)-4-[4'-Methoxy-2',3'-di(methoxymethoxy)phenyl]-5-(3",4",5"-trimethoxyphenyl)-1,3-dioxolane (18). The title compound was prepared by the same reaction conditions described for the synthesis of (*R*,*R*)-1,3-dioxolane **19,** but (*S*,*S*)-diol **16** (2.1 g; 4.7 mmol) was used to yield (*S*,*S*)-1,3-dioxolane **18** as a clear oil (1.8 g; 79%): R_{f} 0.54 (2:1, hexane-EtOAc); [α]_{D}²⁵ -100° (c = 0.46, CHCl₃); TOFMS *m*/*z* 489 (M + Na)⁺; IR (film) vₘₐₓ 2941, 1593, 1498, 1460, 1421, 1157, 1128, 1089, 968, 925 cm⁻¹; ¹H NMR (300 MHz, CDCl₃) δ 3.31 (3H, s, OCH₂OCH₃), 3.55 (3H, s, OCH₂OCH₃), 3.82 (9H, s, 3 x OCH₃), 3.87 (3H, s, OCH₃), 4.86 (1H, d, *J* = 6.0 Hz, CH), 4.91 (1H, d,*J* = 5.4 Hz, OCH₂OCH₃), 5.02 (1H, *d, J =* 5.4 Hz, OCH₂OCH₃), 5.07 (1H, d, *J* = 6.0 Hz, OCH₂OCH₃), 5.10 (1H, d, *J* = 5.4 Hz, OCH₂OCH₃), 5.19 (1H, d, J = 6.6 Hz, CH), 5.39 (1H, s, CH bridgehead), 5.44 (1H, s, CH bridgehead), 6.57 (2H, s, H-2", H-6"), 6.78 (1H, d, J= 8.7 Hz, H-5'), 7.24 (1H, d, J= 9.0 Hz, H-6'). Anal. Calcd. For C₂₃H₃₀O₁₀: C, 59.22%: H, 6.48%. Found: C, 59.30%: H, 6.31%.
**(4*R*,5*R*)-4-[4'-Methoxy-2',3'-di(methoxymethoxy)phenyl]-5-(3",4",5"-trimethoxyphenyl)-1,3-dioxolane (19).** A solution of NaOH (9.2 g; 230 mmol; 75 eq) in H₂O (10 mL) was added over a period of 10 min to a stirred solution of (*R,R*)-diol **17** (1.4 g; 3.1 mmol), dibromomethane (6.5 mL; 92 mmol; 30 eq), cetyltrimethylammonium bromide (0.11 g; 0.29 mmol; 0.09 eq) in CH₂Cl₂ (50 mL), under gentle reflux. After 4.5 hours the light yellow solution was extracted with CH₂Cl₂ (4 x 25 mL), and the combined extract washed with H₂O and dried. The solvent was concentrated *in vacuo* to yield a yellow oil, that was separated by flash column chromatography (eluent 2:1 hexane-EtOAc) to provide dioxolane **19** as a clear oil (0.98 g; 69%): R_{f} 0.51 (2:1, hexane-EtOAc); [α]_{D}²⁵ +91° (c = 0.63, CHCl₃); TOFMS *m*/*z* 488 (M + Na)⁺; IR (film) vₘₐₓ 2941, 1593, 1498, 1456, 1419, 1157, 1126, 1087, 966, 923 cm⁻¹; ¹H NMR (300 MHz, CDCl₃) δ 3.31 (3H, s, OCH₂OCH₃), 3.55 (3H, s, OCH₂OCH₃), 3.82 (9H, s, 3 x OCH₃), 3.87 (3H, s, OCH₃), 4.86 (1H, d, *J* = 6.0 Hz, CH), 4.91 (1H, d, *J* = 5.4 Hz, OCH₂OCH₃), 5.02 (1H, d, *J =* 5.4 Hz, OCH₂OCH₃), 5.07 (1H, d, *J* = 6.0 Hz, OCH₂OCH₃), 5.10 (1H, d, *J* = 5.4 Hz, OCH₂OCH₃), 5.19 (1H, d, *J =* 6.6 Hz, CH), 5.39 (1H, s, CH bridgehead), 5.44 (1H, s, CH bridgehead), 6.58 (2H, s, H-2", H-6"), 6.79 (1H, d, *J* = 8.7 Hz, H-5'), 7.24 (1H, d, *J* = 9.0 Hz, H-6'). Anal. Calcd. For C₂₃H₃₀O₁₀: C, 59.22%: H, 6.48%. Found: C, 59.19%: H, 6.60%.
**(4*S*,5*S*)-4-(2',3'-Dihydroxy-4'-methoxyphenyl)-5-(3",4",5"-trimethoxyphenyl)-1,3-diozolane [dioxostatin] (9a).** To a stirred solution of dimethoxymethyl ether **18** (1.7 g; 3.64 mmol) in THF (10 mL) and IPA (4 mL) was added 6N HCL (6 mL; 36 mmol; 9 eq). After 16 hours the light yellow mixture was extracted with CH₂Cl₂ (4 x 20 mL), and the combined organic extract washed with brine and dried. The solvent was concentrated *in vacuo* to yield a light brown oil that was subjected to column chromatography [gravity column (eluent 49:50:1 hexane-EtOAc-AcOH)] to afford a colorless fluffy solid which was recrystallized from EtOAc-hexane (0.82 g; 59%): m.p. 151-152 °C; R_{f} 0.84 (66:33:1, EtOAc-hexane-AcOH); [α]_{D}²⁵ -68° (c = 1.16, CHCl₃); TOFMS *m*/*z* 399 (M + Na)⁺; IR (film) vₘₐₓ 3452, 3288, 1593, 1510, 1464, 1327, 1292, 1236, 1124, 1093 cm⁻¹; ¹H NMR (300 MHz, CDCl₃) δ 3.81 (6H, s, 2 x OCH₃), 3.84 (3H, s, OCH₃), 3.89 (3H, s, OCH₃), 4.91 (2H, s, 2 x CH), 5.40 (1H, s, CH bridgehead), 5.46 (1H, brs, OH, D₂O exchanged), 5.49 (1H, s, CH bridgehead), 6.46 (1H, d, *J* = 8.1 Hz, H-5'), 6.47 (1H, brs, OH, D₂O exchanged), 6.53 (2H, s, H-2", H-6"), 6.60 (1H, d, *J* = 9.0 Hz, H-6'). Anal. Calcd. For C₁₉H₂₂O₈ • ¼H₂O: C, 59.60%: H, 5.92%. Found: C, 59.81%: H, 6.23%.
**(4*R*,5*R*)-4-(2',3'-Dihydroxy-4'-methoxyphenyl)-5-(3",4",5"-trimethoxyphenyl)-1,3-dioxolane (10a).** Dimethoxymethyl ether **19** (0.90 g; 1.9 mmol) was deprotected (as described above for obtaining diphenol **9a**) to yield **10a** as a colorless fluffy solid (0.29 g; 40%): m.p. 151-152 °C; R_{f} 0.89 (66:33:1, EtOAc-hexane-AcOH);[α]_{D}²⁵ +71° (c = 1.13, CHCl₃); TOFMS *m*/*z* 399 (M + Na)⁺; IR (film) vₘₐₓ 3447, 3284, 1593, 1510, 1464, 1329, 1296, 1236, 1126, 1095 cm⁻¹; ¹H NMR (300 MHz, CDCl₃) δ 3.81 (6H, s, 2 x OCH₃), 3.84 (3H, s, OCH₃) 3.89 (3H, s, OCH₃), 4.91 (2H, s, 2 x CH), 5.39 (1H, s, CH bridgehead), 5.46 (1H, brs, OH, D₂O exchanged), 5.49 (1H, s, CH bridgehead), 6.45 (1H, d, *J*= 8.1 Hz, H-5'), 6.47 (1H, brs, OH, D₂O exchanged), 6.53 (2H, s, H-2", H-6"), 6.60 (1H, d, *J* = 9.0 Hz, H-6'). Anal. Calcd. For C₁₉H₂₂O₈: C, 60.31%: H, 5.86%. Found: C, 59.96%: H, 6.15%.
**(4*S*,5*S*)-4-(2',3'-*O*-Di[bis(benzyl)phosphoryl)-4'-methoxyphenyl)-5-(3",4",5"-trimethoxyphenyl)-1,3-dioxolane (20)**. A solution of diphenol 9a (0.2 g; 0.53 mmol) in acetonitrile (10 mL) was cooled to -20 °C. Carbon tetrachloride (0.6 mL; 5.3 mmol; 10 eq) was added. The resulting solution was stirred for 10 min before The addition of DIPEA (0.4 mL; 2.2 mmol; 4.2 eq, *via* syringe) and DMAP (15 mg; 0.11 mmol; 0.2 eq). Approximately 1 min later, slow (dropwise) addition of dibenzyl phosphite (0.34 mL; 1.5 mmol; 2.9 eq) was begun at such a rate that the reaction temperature was kept below -20 °C. After 45 min of stirring, 0.5 M KH₂PO₄ (aq) was added and the reaction mixture was allowed to warm to room temperature. The mixture was extracted with EtOAc (4 x 15 mL), and the combined organic extract was successively washed with saturated NaCl (aq), water, and dried. Removal of the solvent under reduced pressure afforded a yellow oil that was separated by flash column chromatography (3:2 hexane-EtOAc) to afford 0.43 g (90%) of a clear oil: R_{f} 0.24 (1:1, hexane-EtOAc); [α]_{D}²⁵ -55° (c = 0.83, CHCl₃); EIMS *m*/*z* 898 (5%, M⁺), 778 (10%), 546 (5%), 181 (20%), 91 (100%); IR (film) vₘₐₓ 2944, 1593, 1506, 1458, 129b, 1126, 1099, 1014, 898, 740 cm⁻¹; ¹H NMR (300 MHz, CDCl₃) δ 3.70 (3H, s, OCH₃), 3.76 (6H, s, 2 x OCH₃), 3.81 (3H, s, OCH₃), 4.75-5.11 (8H, m, 4 x CH₂-Ph), 5.16 (1H, d, J= 6.6 Hz, CH), 5.29 (1H, s, CH bridgehead), 5.41 (1H, s, CH bridgehead), 5.50 (1H, d, J= 6.6 Hz, CH), 6.57 (2H, s, H-2", H-6"), 6.91 (1H, d, J= 8.7 Hz, H-5'), 7.43 (1H, d, *J* = 9.3 Hz, H-6'), 7.07-7.25 (15H, m, 4 x C₆H₅), 7.30 (5H, m, C₆H₅); ¹³C NMR (125 MHz, CDCl₃) δ 153.29, 152.13, 137.53, 135.74, 135.35, 133.54, 128.36, 127.84, 127.69, 127.61, 124.48, 123.65, 110.06, 102.94, 95.90, 84.69, 78.73, 77.62, 70.04, 69.67, 60.61, 56.24, 56.01; ³¹P NMR (202 MHz, CDCl₃) δ -4.79, -4.86. Anal. Calcd. For C₄₇H₄₈O₁₄P₂: C, 62.81%: H, 5.38%. Found: C, 62.72%: H, 5.34%.
**Tetrasodium-(4*S*,5*S*)-4-(2',3'-*O*-diphosphonate-4'-methoxyphenyl)-5-(3",4",5"-trimethoxyphenyl)-1,3-dioxolane (21)**. To a solution of tetrabenzyl diphosphate **20** (0.43 g; 0.45 mmol) in EtOH (5 mL in a hydrogenation flask) was added 10% Pd/C (0.14 g). The flask was evacuated and exposed (balloon) to a hydrogen atmosphere for 2 hours. Filtration of the solution through celite and subsequent evaporation (*in* *vacuo*) of solvent afforded a clear oil. Anhydrous MeOH (5 mL) was added to the crude phosphoric acid followed by NaOCH₃ (0.11 g; 1.9 mmol; 4 eq). The mixture was stirred for 12 hours, at which point additional MeOH was added until the product dissolved. Collection (filtration) of the product in MeOH and subsequent concentration *in vacuo* afforded a crude colorless solid, which was reprecipitated from H₂O/EtOH to yield a colorless powder (0.22 g; 72%): m.p. 166-168 °C dec.; [α]_{D}²⁵ - 68° (c=1.05, H₂O); UV λₘₐₓ (H₂O) 285 nm (log ε, 3.17); IR (KBr) νₘₐₓ 3375, 2339, 1591, 1496, 1454, 1296, 1168, 1120, 1095, 985 cm⁻¹; ¹H NMR (500 MHz, D₂O) δ 3.59 (3H, s, OCH₃), 3.68 (3H, s, OCH₃), 3.71 (6H, s, 2 x OCH₃), 5.04 (1H, d, *J* = 5.5 Hz, CH), 5.15 (1H, s, CH bridgehead), 5.18 (1H, s, bridgehead), 5.85 (1H, d, *J* = 4.0 Hz, CH), 6.68 (1H, d, *J* = 8.5 Hz, H-5'), 6.71 (2H, s, H-2", H-6"), 7.03 (1H, d, *J* = 8.5 Hz, H-6'); ¹³C NMR (125 MHz, D₂O/CD₃OD) δ 154.69, 153.70, 147.42, 137.69, 135.86, 124.15, 122.09, 108.26, 105.53, 95.55, 83.19, 79.75, 61.68, 57.17, 56.74. ³¹P NMR (202 MHz, D₂O) δ 1.79, 1.97. LRFAB MS *m*/*z* 603 [(Anion + 3Na)⁻, 25%], 581 [(Anion +2Na +H)⁻, 70%], 559 [(Anion + Na + 2H)⁻, 100%].

Tubulin assays. The tubulin polymerization and colchicine binding experiments were performed as described previously (See: Verdier-Pinard, P.; Lai, J-Y.; Yoo, H-D.; Yu, J.; Marquez, B.; Nagle, D.G.; Nambu, M.; White, J.D.; Falck, J.R.; Gerwick, W.H.; Day, B.W.; Hamel, E. Mol. Pharmacol. **1998**, *53*, 62-76). However, in the polymerization assays Beckman DU7400/7500 spectrophotometers equipped with "high performance" temperature controllers were used. Unlike the manual control possible with the previously used Gilford spectrophotometers, the Beckman instruments are microprocessor controlled, and assays required use of programs provided by MDB Analytical Associates, South Plainfield, NJ. The Beckman instruments were unable to maintain 0°C and the lower temperature in the assays fluctuated between 2 and 4°C. Temperature changes were, however, more rapid, with the jump from the lower temperature to 30°C taking about 20 sec and the reverse jump about 100 sec.

Antimicrobial susceptibility testing. Compounds were screened against the bacteria *Stenotrophomonas maltophilia, Micrococcus luteus, Staphylococcus aureus, Escherichia coli, Enterobacter cloacae, Enterococcus faecalis, Streptococcus pneumoniae, Neisseria gonorrhoeae*, and the fungi *Candida albicans* and *Cryptococcus neoformans*, according to established disk susceptibility testing protocols (See: Oshumi, K.; Nakagawa, R.; Yumiko, F.; Hatanaka, T.; Morinaga, Y.; Nihei, Y.; Obishi, D.; Suga, Y.; Akiyama, Y.; Tsuji, T. Novel Combretastatin Analogues Effective Against Murine Solid Tumors: Design and Structure-Activity Relationships. J. Med. Chem. **1998**, *41*, 3022-3032; Madarde, M.; Ramos, a.; Caballero, E.; Pelaez-Lamamie de Clairac, R.; Lopez, J.L.; Gravalos, D.G.; Feliciano, A.S. Synthesis and Antineoplastic Activity of Combretastatin Analogues: Heterocombretastatins. Eur. J. Med. Chem. **1998**, *33*, 71-77).

### Dosages

The dosage administered will be dependent upon the identity of the neoplastic disease; the type of host involved, including its age, health and weight; the kind of concurrent treatment, if any; the frequency of treatment and therapeutic ratio.

Illustratively, dosage levels of the administered active ingredients are: intravenous, 0.1 to about 200 mg/kg; intramuscular, 1 to about 500 mg/kg; orally, 5 to about 1000 mg/kg; intranasal instillation, 5 to about 1000 mg/kg; and aerosol, 5 to about 1000 mg/k of host body weight.

Expressed in terms of concentration, an active ingredient can be present in the compositions of the present invention for localized use about the cutis, intranasally, pharyngolaryngeally, bronchially, intravaginally, rectally, or ocularly in concentration of from about 0.01 to about 50% w/w of the composition; preferably about 1 to about 20% w/w of the composition; and for parenteral use in a concentration of from about 0.05 to about 50% w/v of the composition and preferably from about 5 to about 20% w/v.

The compositions of the present invention are preferably presented for administration to humans and animals in unit dosage forms, such as tablets, capsules, pills, powders, granules, suppositories, sterile parenteral solutions or suspensions, sterile non-parenteral solutions of suspensions, and oral solutions or suspensions and the like, containing suitable quantities of an active ingredient.

For oral administration either solid or fluid unit dosage forms can be prepared.

Powders are prepared quite simply by comminuting the active ingredient to a suitably fine size and mixing with a similarly comminuted diluent. The diluent can be an edible carbohydrate material such as lactose or starch. Advantageously, a sweetening agent or sugar is present as well as a flavoring oil.

Capsules are produced by preparing a powder mixture as hereinbefore described and filling into formed gelatin sheaths. Advantageously, as an adjuvant to the filling operation, a lubricant such as talc, magnesium stearate, calcium stearate and the like is added to the powder mixture before the filling operation.

Soft gelatin capsules are prepared by machine encapsulation of a slurry of active ingredients with an acceptable vegetable oil, light liquid petrolatum or other inert oil or triglyceride.

Tablets are made by preparing a powder mixture, granulating or slugging, adding a lubricant and pressing into tablets. The powder mixture is prepared by mixing an active ingredient, suitably comminuted, with a diluent or base such as starch, lactose, kaolin, dicalcium phosphate and the like. The powder mixture can be granulated by wetting with a binder such as corn syrup, gelatin solution, methylcellulose solution or acacia mucilage and forcing through a screen. As an alternative to granulating, the powder mixture can be slugged, i.e., run through the tablet machine and the resulting imperfectly formed tablets broken into pieces (slugs). The slugs can be lubricated to prevent sticking to the tablet-forming dies by means of the addition of stearic acid, a stearic salt, talc or mineral oil. The lubricated mixture is then compressed into tablets.

Advantageously, the tablet can be provided with a protective coating consisting of a sealing coat or enteric coat of shellac, a coating of sugar and methylcellulose and polish coating of camauba wax.

Fluid unit dosage forms for oral administration such as in syrups, elixirs and suspensions can be prepared wherein each teaspoonful of composition contains a predetermined amount of an active ingredient for administration. The water-soluble forms can be dissolved in an aqueous vehicle together with sugar, flavoring agents and preservatives to form a syrup. An elixir is prepared by using a hydroalcoholic vehicle with suitable sweeteners together with a flavoring agent. Suspensions can be prepared of the insoluble forms with a suitable vehicle with the aid of a suspending agent such as acacia, tragacanth, methylcellulose and the like.

For parenteral administration, fluid unit dosage forms are prepared utilizing an active ingredient and a sterile vehicle, water being preferred. The active ingredient, depending on the form and concentration used, can be either suspended or dissolved in the vehicle. In preparing solutions the water-soluble active ingredient can be dissolved in water for injection and filter sterilized before filling into a suitable vial or ampule and sealing. Advantageously, adjuvants such as a local anesthetic, preservative and buffering agents can be dissolved in the vehicle. Parenteral suspensions are prepared in substantially the same manner except that an active ingredient is suspended in the vehicle instead of being dissolved and sterilization cannot be accomplished by filtration. The active ingredient can be sterilized by exposure to ethylene oxide before suspending in the sterile vehicle. Advantageously, a surfactant or wetting agent is included in the composition to facilitate uniform distribution of the active ingredient.

In addition to oral and parenteral administration, the rectal and vaginal routes can be utilized. An active ingredient can be administered by means of a suppository. A vehicle which has a melting point at about body temperature or one that is readily soluble can be utilized. For example, cocoa butter and various polyethylene glycols (Carbowaxes) can serve as the vehicle.

For intranasal instillation, a fluid unit dosage form is prepared utilizing an active ingredient and a suitable pharmaceutical vehicle, preferably P.F. water; a dry powder can be formulated when insufflation is the administration of choice.

For use as aerosols, the active ingredients can be packaged in a pressurized aerosol container together with a gaseous or liquefied propellant, for example, dichlorodifluoromethane, carbon dioxide, nitrogen, propane, and the like, with the usual adjuvants such as cosolvents and wetting agents, as may be necessary or desirable.

The term "unit dosage form" as used in the specification and claims refers to physically discrete units suitable as unitary dosages for human and animal subjects, each unit containing a predetermined quantity of active material calculated to produce the desired therapeutic effect in association with the required pharmaceutical diluent, carrier or vehicle. The specifications for the novel unit dosage forms of this invention are dictated by and are directly dependent on (a) the unique characteristics of the active material and the particular therapeutic effect to be achieved, and (b) the limitation inherent in the art of compounding such an active material for therapeutic use in humans, as disclosed in this specification, these being features of the present invention. Examples of suitable unit dosage forms in accord with this invention are tablets, capsules, troches, suppositories, powder packets, wafers, cachets, teaspoonfuls, tablespoonfuls, dropperfuls, ampules, vials, segregated multiples of any of the foregoing, and other forms as herein described.

The active ingredients to be employed as antineoplastic agents can be easily prepared in such unit dosage form with the employment of pharmaceutical materials which themselves are available in the art and can be prepared by established procedures. The following preparations are illustrative of the preparation of the unit dosage forms of the present invention, and not as a limitation thereof. Several dosage forms were prepared embodying the present invention. They are shown in the following examples in which the notation "active ingredient" signifies either dioxostatin (9a) and/or dioxostatin prodrug or any other compound described herein.

### COMPOSITION "A"

### Hard-Gelatin Capsules

One thousand two-piece hard gelatin capsules for oral use, each capsule containing 200 mg of an active ingredient are prepared from the following types and amounts of ingredients:

| | |
|---|---|
| Active ingredient, micronized | 200 g |
| Corn Starch | 20 g |
| Talc | 20 g |
| Magnesium stearate | 2 g |

The active ingredient, finely divided by means of an air micronizer, is added to the other finely powdered ingredients, mixed thoroughly and then encapsulated in the usual manner.

The foregoing capsules are useful for treating a neoplastic disease by the oral administration of one or two capsules one to four times a day.

Using the procedure above, capsules are similarly prepared containing an active ingredient in 50, 250 and 500 mg amounts by substituting 50 g, 250 g and 500 g of an active ingredient for the 200 g used above.

### COMPOSITION "B"

### Soft Gelatin Capsules

One-piece soft gelatin capsules for oral use, each containing 200 mg of an active ingredient, finely divided by means of an air micronizer, are prepared by first suspending the compound in 0.5 ml of corn oil to render the material capsulatable and then encapsulating in the above manner.

The foregoing capsules are useful for treating a neoplastic disease by the oral administration of one or two capsules one to four times a day.

### COMPOSITION "C"

### Tablets

One thousand tablets, each containing 200 mg of an active ingredient, are prepared from the following types and amounts of ingredients:

| | |
|---|---|
| Active ingredient, micronized | 200 g |
| Lactose | 300 g |
| Com starch | 50 g |
| Magnesium stearate | 4 g |
| Light liquid petrolatum | 5 g |

The active ingredient, finely divided by means of an air micronizer, is added to the other ingredients and then thoroughly mixed and slugged. The slugs are broken down by forcing them through a Number Sixteen screen. The resulting granules are then compressed into tablets, each tablet containing 200 mg of the active ingredient.

The foregoing tablets are useful for treating a neoplastic disease by the oral administration of one or two tablets one to four times a day.

Using the procedure above, tablets are similarly prepared containing an active ingredient in 250 mg and 100 mg amounts by substituting 250 g and 100 g of an active ingredient for the 200 g used above.

### COMPOSITION "D"

### Oral Suspension

One liter of an aqueous suspension for oral use, containing in each teaspoonful (5 ml) dose, 50 mg of an active ingredient, is prepared from the following types and amounts of ingredients:

| | |
|---|---|
| Active ingredient, micronized | 10 g |
| Citric acid | 2 g |
| Benzoic acid | 1 g |
| Sucrose | 790 g |
| Tiagacanth | 5 g |
| Lemon Oil | 2 g |
| Deionized water, q.s. 1000 ml | |

The citric acid, benzoic acid, sucrose, tragacanth and lemon oil are dispersed in sufficient water to make 850 ml of suspension. The active ingredient, finely divided by means of an air micronizer, is stirred into the syrup unit uniformly distributed. Sufficient water is added to make 1000 ml.

The composition so prepared is useful for treating a neoplastic disease at a dose of 1 teaspoonful (15 ml) three times a day.

### COMPOSITION "E"

### Parenteral Product

A sterile aqueous suspension for parenteral injection, containing 30 mg of an active ingredient in each milliliter for treating a neoplastic disease, is prepared from the following types and amounts of ingredients:

| | |
|---|---|
| Active ingredient, micronized | 30 g |
| POLYSORBATE 80 | 5 g |
| Methylparaben | 2.5 g |
| Propylparaben | 0.17 g |
| Water for injection, q.s. 1000 ml. | |

All the ingredients, except the active ingredient, are dissolved in the water and the solution sterilized by filtration. To the sterile solution is added the sterilized active ingredient, finely divided by means of an air micronizer, and the final suspension is filled into sterile vials and the vials sealed.

The composition so prepared is useful for treating a neoplastic disease at a dose of I milliliter (1ml) three times a day.

### COMPOSITION "F"

### Suppository, Rectal and Vaginal

One thousand suppositories, each weighing 2.5 g and containing 200 mg of an active ingredient are prepared from the following types and amounts of ingredients:

| | |
|---|---|
| Active ingredient, micronized | 15 g |
| Propylene glycol | 150 g |
| Polyethylene glycol #4000, q.s. | 2,500 g |

The active ingredient is finely divided by means of an air micronizer and added to the propylene glycol and the mixture passed through a colloid mill until uniformly dispersed. The polyethylene glycol is melted and the propylene glycol dispersion is added slowly with stirring. The suspension is poured into unchilled molds at 40°C. The composition is allowed to cool and solidify and then removed from the mold and each suppository foil wrapped.

The foregoing suppositories are inserted rectally or vaginally for treating a neoplastic disease.

### COMPOSITION "G"

### Intranasal Suspension

One liter of a sterile aqueous suspension for intranasal instillation, containing 20 mg of an active ingredient in each milliliter, is prepared from the following types and amounts of ingredients:

| | |
|---|---|
| Active ingredient, micronized | 15 g |
| POLYSORBATE 80 | 5g |
| Methylparaben | 2.5 g |
| Propylparaben | 0.17 g |
| Deionized water, q.s. 1000 ml. | |

All the ingredients, except the active ingredient, are dissolved in the water and the solution sterilized by filtration. To the sterile solution is added the sterilized active ingredient, finely divided by means of an air micronizer, and the final suspension is aseptically filled into sterile containers.

The composition so prepared is useful for treating a neoplastic disease, by intranasal instillation of 0.2 to 0.5 ml given one to four times per day.

An active ingredient can also be present in the undiluted pure form for use locally about the cutis, intranasally, pharyngolaryngeally, bronchially, or orally.

### COMPOSITION "H"

### Powder

Five grams of an active ingredient in bulk form is finely divided by means of an air micronizer. The micronized powder is placed in a shaker-type container.

The foregoing composition is useful for treating a neoplastic disease, at localized sites by applying a powder one to four times per day.

### COMPOSITION "I"

### Oral Powder

One hundred grams of an active ingredient in bulk form is finely divided by means of an air micronizer. The micronized powder is divided into individual doses of 200 mg and packaged.

The foregoing powders are useful for treating a neoplastic disease, by the oral administration of one or two powders suspended in a glass of water, one to four times per day.

### COMPOSITION "J"

### Insufflation

One hundred grams of an active ingredient in bulk form is finely divided by means of an air micronizer.

The foregoing composition is useful for treating a neoplastic disease, by the inhalation of 300 mg one to four times a day.

From the foregoing, it becomes readily apparent that a new and useful antineoplastic factor and new and useful antineoplastic preparations and microtuble inhibitors have been herein described and illustrated which fulfill all of the aforestated objectives in a remarkably unexpected fashion. h is of course understood that such modifications, alterations and adaptations as will readily occur to the artisan confronted with this disclosure are intended within the spirit of the present invention.

## Claims

1. A compound denominated "dioxostatin", having the general structure set forth below:

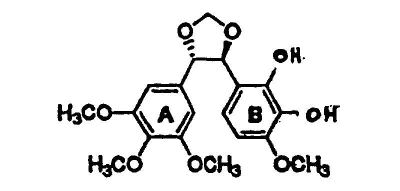

2. A compound according to Claim 1 for use in medicine.

3. Use of a compound according to Claim 1 in the manufacture of a medicament for treating cells afflicted with a neoplastic disease.

4. A method of inhibiting microtubule assembly in an in vitro sample comprising exposing said microtubules to an effective amount of the active ingredient dioxostatin in a pharmacologically acceptable carrier.

5. Use of a compound according to Claim 1 in the manufacture of a medicament for inhibiting microtubule assembly.

6. A pharmaceutical composition comprising a compound according to Claim 1 and a pharmacological acceptable carrier.

7. A pharmaceutical composition according to Claim 6 in which said carrier comprises an aqueous solution.

## Patentansprüche

1. "Dioxostatin" bezeichnete Verbindung mit der Grundstruktur, die unten dargestellt ist:

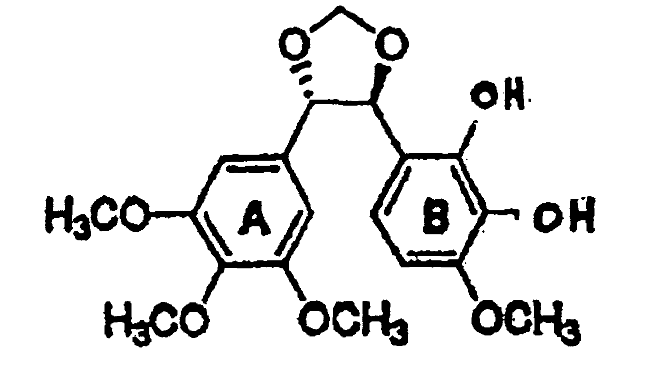

2. Verbindung nach Anspruch 1 zur Verwendung in der Medizin.

3. Verwendung einer Verbindung nach Anspruch 1 bei der Herstellung eines Medikaments zur Behandlung von Zellen, die von einer neoplastischen Erkrankung betroffen sind.

4. Verfahren zum Verhindern einer Mikroröhrchenansammlung bei einer in vitro Probe, das das Aussetzen der Mikroröhrchen gegenüber einer wirksamen Menge des aktiven Inhaltsstoffs Dioxostatin in einem pharmazeutisch akzeptablen Träger umfasst.

5. Verwendung einer Verbindung nach Anspruch 1 bei der Herstellung eines Medikaments zur Verhinderung einer Mikroröhrchenansammlung.

6. Pharmazeutische Zusammensetzung mit einer Verbindung nach Anspruch 1 und einem pharmazeutisch akzeptablen Träger.

7. Pharmazeutische Zusammensetzung nach Anspruch 6, wobei der Träger eine wässrige Lösung aufweist.

## Revendications

1. Composé dénommé "dioxostatine", ayant la structure générale indiquée ci-dessous :

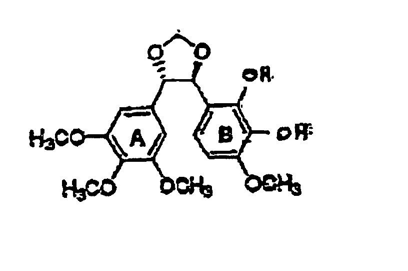

2. Composé selon la revendication 1, pour utilisation en médecine.

3. Utilisation d'un composé selon la revendication 1, dans la fabrication d'un médicament destiné à traiter des cellules frappées d'une maladie néoplasique.

4. Procédé d'inhibition d'un assemblage de microtubules dans un échantillon in vitro comprenant l'exposition desdites microtubules à une quantité efficace de l'ingrédient actif dioxostatine dans un véhicule pharmaceutiquement acceptable.

5. Utilisation d'un composé selon la revendication 1, dans la fabrication d'un médicament pour inhiber un assemblage de microtubules.

6. Composition pharmaceutique comprenant un composé selon la revendication 1 et un véhicule pharmaceutiquement acceptable.

7. Composition pharmaceutique selon la revendication 6, dans laquelle ledit véhicule comprend une solution aqueuse.
